Europäisches Patentamt

European Patent Office

Office européen des brevets

(19)

(11) Veröffentlichungsnummer: **0 121 874**
**B1**

(12) **EUROPÄISCHE PATENTSCHRIFT**

(45) Veröffentlichungstag der Patentschrift:
17.09.86

(21) Anmeldenummer: 84103517.3

(22) Anmeldetag: 30.03.84

(51) Int. Cl.⁴: **C 08 F 226/10**, C 08 F 218/04,
C 08 F 220/28, A 61 K 47/00,
A 61 K 7/11 // (C08F226/10,
218:04, 220:28),(C08F220/28,
218:04, 226:10),(C08F218/04,
226:10, 220:28)

(54) Wasserlösliche Polymere mit geringer Hygroskopie.

(30) Priorität: 08.04.83 DE 3312668

(43) Veröffentlichungstag der Anmeldung:
17.10.84 Patentblatt 84/42

(45) Bekanntmachung des Hinweises auf die Patenterteilung:
17.09.86 Patentblatt 86/38

(84) Benannte Vertragsstaaten:
AT BE CH DE FR GB IT LI NL

(56) Entgegenhaltungen:
DE - A - 2 022 264
GB - A - 1 566 249

(73) Patentinhaber: BASF Aktiengesellschaft,
Carl-Bosch-Strasse 38, D-6700 Ludwigshafen (DE)

(72) Erfinder: Straub, Ferdinand, Dr., Ziegelstrasse 20,
D-6832 Hockenheim (DE)
Erfinder: Sanner, Axel, Dr., Lorscher Ring 2 c,
D-6710 Frankenthal (DE)
Erfinder: Seib, Karl, Dr., Krienhildstrasse 36,
D-6940 Weinheim (DE)
Erfinder: Lang, Siegfried, Dr., Thomas-Mann-Strasse 22,
D-6700 Ludwigshafen (DE)

**Beschreibung**

Die Erfindung betrifft Vinylpyrrolidon (VP)-Copolymerisate, die einerseits wasserlöslich, andererseits nur schwach hygroskopisch sind und daher in feuchter Luft nicht dazu neigen, klebrig zu werden.

Es gibt Fälle, beispielsweise bei Binder- und Überzugsmitteln für Tabletten, in denen von einem Polymeren zwei einander entgegensetzte Eigenschaften verlangt werden, nämlich einerseits Wasserlöslichkeit und andererseits möglichst geringe Hygroskopie, d.h. möglichst geringe Tendenz, im trockenen Zustand aus der Luft Feuchtigkeit aufzunehmen.

Da beide Forderungen sich schwer gleichzeitig erfüllen lassen, verzichtet man in vielen Fällen notgedrungen auf die Wasserlöslichkeit und behilft sich mit anderen Lösungsmitteln oder Lösungsmittelgemischen (vgl. z.B. US 3,445,566; US 4,012,501; US 3,927,199 oder man dispergiert die Stoffe in Wasser (vgl. z.B. US 3,112,215).

Vinylpyrrolidon-Copolymerisate werden in Pharmazie und Kosmetik z.B. als Tablettenbindemittel, Überzugsmittel, Filmbildner eingesetzt. Nachteilig dabei ist die Hygroskopie des Polyvinylpyrrolidons (PVP); PVP-Homopolymerisate nehmen bei 75% rel. Luftfeuchte und 23°C über 26% Wasser auf. Da wasserhaltiges PVP klebt, führt das hygroskopische Verhalten des PVP zu einem unerwünschten Verkleben von Tabletten usw. in feuchten Räumen bzw. Regionen. Durch Copolymerisation von Vinylpyrrolidon, beispielsweise mit Vinylestern, oder durch Zumischen von wasserabstossenden, unpolaren Stoffen, wie Talkum oder Stearylalkohol, wurde schon versucht, das Verkleben zu verhindern. Beim Einpolymerisieren oder Zumischen zu grösser Mengen wasserabstossender Substanzen ist das Copolymerisat bzw. die Abmischung aber nicht mehr wasserlöslich. Die wasserunlöslichen Stoffe setzen sich ab und bringen technische Probleme.

Die bisher bekannten Copolymeren von Vinylpyrrolidon mit Vinylacetat sind nur bei Gehalten von über 60 Gew.-% VP noch wasserlöslich und haben dabei eine Wasseraufnahme von über 18% bei 75% rel. Luftfeuchte und 23°C.

Der Erfindung lag die Aufgabe zugrunde, ein Polymeres zu entwickeln, das gut in Wasser löslich ist und nicht dazu neigt, an feuchter Luft klebrig zu werden.

Erfindungsgemäss wird diese Aufgabe gelöst durch Terpolymerisate folgender Zusammensetzung:

10 bis 40, vorzugsweise 30 bis 40 Gew.-% Vinylpyrrolidon

20 bis 50, vorzugsweise 30 bis 50 Gew.-% Vinylacetat oder Vinylpropionat und

10 bis 40, vorzugsweise 30 bis 35 Gew.-% Hydroxyethylacrylat, Hydroxypropylacrylat oder Hydroxyethylmethacrylat,

mit etwa statistischer Verteilung der Komponenten.

Copolymerisate dieser Zusammensetzung sind (bei richtiger Copolymerisation) leicht wasserlöslich, haben eine geringe Wasseraufnahme (weniger als 15 Gew.-% bei 23°C und 75% rel. Luftfeuchte) und neigen daher nicht dazu, an feuchter Luft klebrig zu werden.

Unter «richtiger Copolymerisation» ist eine solche Führung der Copolymerisation zu verstehen, dass die Zusammensetzung des Polymerisates trotz der starken Verschiedenheit der Copolymerisationsparameter der Komponenten (am langsamsten copolymerisieren die Vinylester, schneller das Vinylpyrrolidon und am schnellsten die Acrylate) nicht nur brutto den eingesetzten Monomeranteilen entspricht, sondern auch über die gesamte Länge der Polymerketten einigermassen gleichmässig ist. Man erreicht dies bekanntlich dadurch, dass man die Zugabegeschwindigkeit entweder aller oder mindestens der rasch copolymerisierenden Komponenten in den Reaktionsraum an die Copolymerisationsgeschwindigkeit der langsamsten Komponenten anpasst, so dass diese sich nicht oder nur geringfügig gegen Ende der Polymerisation anreichern. Ohne diese Massnahme erhielte man kein echtes Copolymerisat (mit etwa statistischer Verteilung der Komponenten), sondern quasi ein Blockcopolymerisat. Ein solches hätte nicht die vorteilhaften Eigenschaften der erfindungsgemässen Copolymerisate, sie würden sich also nicht durch die Kombination von Wasserlöslichkeit und geringer Feuchtigkeitsaufnahme aus der Luft auszeichnen.

Die erfindungsgemässen Terpolymerisate eignen sich zum Einsatz in der Kosmetik und Pharmazie, beispielsweise als Haarfestiger, als Binde- und/oder Überzugsmittel für Tabletten, zur Erzielung einwandfreier Überzüge in den üblichen Filmcoating-Apparaturen bzw. in den üblichen Granuliergeräten.

Die in den Beispielen genannten Teile und Prozente beziehen sich auf das Gewicht.

*Beispiel 1*

In einem Rührkolben mit Rückflusskühler und zwei Tropftrichtern werden je 10% der Mischungen A und B aus

A) 210 Teilen Vinylpyrrolidon, 280 Teilen Vinylacetat, 210 Teilen Hydroxyethylacrylat, 250 Teilen Isopropanol und

B) 5 Teilen tert.-Butylperpivalat und 150 Teilen Isopropanol

vorgelegt und zum schwachen Sieden bei 60°C aufgeheizt. Nach dem Anpolymerisieren werden über die Tropftrichter die restlichen Monomeren (90% A) in 7 Stunden, das restliche Peroxid (90% B) in etwa 9 Stunden zugegeben, wobei die Innentemperatur auf 80°C steigt. Nach weiteren 2 Stunden Kochen unter Rückfluss wird das Lösungsmittel (Isopropanol) mit Wasserdampf abgetrieben, die wässrige Polymerlösung wird gefriergetrocknet. Das Festprodukt ist leicht wasserlöslich, hat einen K-Wert nach H. Fikentscher (Cellulose-Chemie <u>13</u> (1932), Seiten 58 bis 64 und 71 bis 74), gemessen in 2%iger wässriger Lösung bei 20°C von 20 und eine Wasseraufnahme bei 75% rel. Luftfeuchte und 23°C von 13,1%.

*Beispiele 2 bis 5*

Die Polymerisation erfolgt analog Beispiel 1. Bei der in der folgenden Tabelle gezeigten Zusammensetzung erhält man leicht wasserlösliche Polymerisate mit folgender Wasseraufnahme aus der Luft:

| Nr. | Vinylpyrrolidon | | Vinylacetat | | Hydroxyalkyl-Acrylat | | % Wasseraufnahme bei 75% rel. Luftfeuchte und 23°C |
|---|---|---|---|---|---|---|---|
| | Teile | % | Teile | % | Teile | % | |
| 2 | 245 | 35 | 210 | 30 | 245 HEMA | 35 | 10 |
| 3 | 280 | 40 | 210 | 30 | 245 HPA | 30 | 14,8 |
| 4 | 230 | 33 | 230 | 33 | 230 HPA | 33 | 12,8 |
| 5 | 210 | 30 | 280 | 40 | 210 HPA | 30 | 11 |

HEMA = Hydroxyethylmethacrylat
HPA = Hydroxypropylacrylat

### Anwendungsbeispiel

#### Filmcoating

#### Beispiel 6

5    Teile Polymerisat nach Beispiel 5
1,5  Teile eines handelsüblichen roten Farblackes bestehend aus einem basischen Aluminium-salz von 2,4,5,7-Tetrajodfluorescein
5,0  Teile Talkum
88,5 Teile Wasser.

1250 Teile einer wässrigen Suspension der obengenannten Zusammensetzung werden auf 5000 Teile Tablettenkerne aufgesprüht und getrocknet. Man erhält glatte, glänzende Filmtabletten. Die Suspension kann bei hohem Durchsatz kontinuierlich aufgesprüht werden. Es ist nicht notwendig, einen Weichmacher zuzusetzen.

Die Sprühbedingungen: 45°C Zuluft, 2 bar Sprühdruck, kontinuierliches Aufsprühen in einem Laborgerät, und zwar 100 g Suspension in 25 min.

#### Beispiel 7a

Analog Beispiel 6 kann folgende Rezeptur benutzt werden:
5    Teile des Polymerisates nach Beispiel 4
4    Teile Polyethylenglykol vom Molgewicht 6000
0,5  Teile Glycerin
1,5  Teile des Farblackes von Beispiel 6
3,0  Teile Titandioxid
5,0  Teile Talkum
81,0 Teile Wasser.

#### Beispiel 7b

5    Teile Polymerisat nach Beispiel 5
1,5  Teile Polyethylenglykol vom Molgewicht 6000
93,5 Teile Wasser.

#### Haarfestiger

#### Beispiel 8

3 Teile Terpolymerisat nach Beispiel 5
97 Teile dest. Wasser.

#### Beispiel 9

3    Teile Terpolymerisat nach Beispiel 4
0,5  Teile einer 40%igen wässrigen Lösung eines Copolymerisates aus 95 Gew.-% Vinylimid-azolium-Methochlorid und 5 Gew.-% Vinyl-pyrrolidon
96,5 Teile dest. Wasser.

#### Beispiel 10

3,0  Teile Terpolymerisat nach Beispiel 4
30,0 Teile Isopropanol
67,0 Teile Wasser dest.

#### Beispiel 11

3,0  Teile Terpolymerisat nach Beispiel 5
0,5  Teile der gleichen 40%igen wässrigen Co-polymerlösung wie bei Beispiel 9
30,0 Teile Ethanol abs.
66,5 Teile Wasser dest.

#### Fönlotions

#### Beispiel 12

0,7  Teile Terpolymerisat nach Beispiel 4
99,3 Teile dest. Wasser.

#### Beispiel 13

0,7  Teile Terpolymerisat nach Beispiel 5
0,5  Teile der gleichen 40%igen wässrigen Co-polymerisatlösung wie bei Beispiel 9
98,8 Teile dest. Wasser.

#### Beispiel 14

0,7  Teile Terpolymerisat nach Beispiel 5
30,0 Teile Isopropanol
69,3 Teile Wasser dest.

#### Beispiel 15

0,7  Teile Terpolymerisat nach Beispiel 4
0,5  Teile der gleichen 40%igen wässrigen Co-polymerlösung wie bei Beispiel 9
30,0 Teile Ethanol abs.
68,8 Teile Wasser dest.

#### Haarsprays

#### Beispiel 16

2,0  Teile Terpolymerisat nach Beispiel 4
38,0 Teile Ethanol abs.
60,0 Teile eines Gemisches von $CFCl_3/CF_2Cl_2$ im Gew.-Verhältnis 50/50.

#### Beispiel 17

2,0  Teile Terpolymerisat nach Beispiel 5
35,0 Teile Methylenchlorid
33,0 Teile Isopropanol
30,0 Teile Propan/Butan im Gew.-Verhältnis 40/60.

*Beispiel 18*

2,0   Teile Terpolymerisat nach Beispiel 4
35,0   Teile Methylenchlorid
13,0   Teile Ethanol abs.
30,0   Teile Propan/Butan 40/60.

*Granulierungsversuche*

*Beispiel 19*

I   { 90    Teile Al-Salz der Acetylsalicylsäure
     { 75    Teile Mannit
     { 10    Teile Maisstärke

II   7     Teile Polymer von Beispiel 5 (gelöst in 30 Teilen $H_2O$)

     1,8    Teile Schmiermittel (Mischung aus 1 Teil Aerosil, 1 Teil Ca-Arachinat und 8 Teilen Talkum).

Mischung I wird mit II intensiv befeuchtet und durch ein Sieb mit Maschenweite 0,5 mm gegeben und getrocknet.

Nach dem Trocknen wird erneut gesiebt und mit 1,8 Teilen Schmiermittel gemischt.

Man erhält beim Tablettieren dieses Granulats Tabletten mit hoher Festigkeit und gutem Zerfall.

## Patentansprüche

1. Wasserlösliches Terpolymerisat aus
a) 10 bis 40 Gew.-% Vinylpyrrolidon,
b) 20 bis 50 Gew.-% Vinylacetat (VAc) oder Vinylpropionat und
c) 10 bis 40 Gew.-% Hydroxyethylacrylat, Hydroxypropylacrylat oder Hydroxyethylmethacrylat, mit etwa statistischer Verteilung der Komponenten.

2. Wasserlösliches Terpolymerisat nach Anspruch 1 mit folgender Zusammensetzung
a) 30 bis 40 Gew.-% Vinylpyrrolidon,
b) 30 bis 40 Gew.-% Vinylacetat oder Vinylpropionat,
c) 30 bis 35 Gew.-% Hydroxyethylacrylat, Hydroxypropylacrylat oder Hydroxyethylmethacrylat.

3. Verwendung eines Terpolymerisates nach Anspruch 1 oder 2 als Filmbildner und Überzugsmittel in Kosmetik und Pharmazie.

## Claims

1. A water-soluble terpolymer which consists of
a) from 10 to 40% by weight of vinylpyrrolidone,
b) from 20 to 50% by weight of vinyl acetate (VAc) or vinyl propionate, and
c) from 10 to 40% by weight of hydroxyethyl acrylate, hydroxypropyl acrylate or hydroxyethyl methacrylate, the components being in substantially random distribution.

2. A water-soluble terpolymer as claimed in claim 1, which has the following composition:
a) from 30 to 40% by weight of vinylpyrrolidone,
b) from 30 to 40% by weight of vinyl acetate or vinyl propionate, and
c) from 30 to 35% by weight of hydroxyethyl acrylate, hydroxypropyl acrylate or hydroxyethyl methacrylate.

3. The use of a terpolymer as claimed in claim 1 or 2 as a film-former or coating material in the cosmetics and pharmaceuticals fields.

## Revendications

1. Terpolymérisat soluble dans l'eau, de
a) 10 à 40% en poids de vinylpyrrolidone,
b) 20 à 50% en poids d'acétate de vinyle (VAC) ou propionate de vinyle et
c) 10 à 40% en poids d'acrylate d'hydroxyéthyle, acrylate d'hydroxypropyle ou méthacrylate d'hydroxyéthyle, à distribution à peu près statistique des composants.

2. Terpolymérisat soluble dans l'eau, selon la revendication 1, de la composition suivante:
a) 30 à 40% en poids de vinylpyrrolidone,
b) 30 à 40% en poids d'acétate de vinyle ou de propionate de vinyle,
c) 30 à 35% en poids d'acrylate d'hydroxyéthyle, acrylate d'hydroxypropyle ou méthacrylate d'hydroxyéthyle.

3. Utilisation d'un terpolymérisat selon la revendication 1 ou 2 comme filmogène et matière d'enrobage en cosmétique et pharmacie.